# EUROPÄISCHE PATENTSCHRIFT

(11) **EP 2 129 216 B1**
(45) Veröffentlichungstag und Bekanntmachung des Hinweises auf die Patenterteilung: **17.08.2011**
(21) Anmeldenummer: 08734901.5
(22) Anmeldetag: 31.03.2008
(51) Int. Cl.: A01N 1/02, A61K 31/192, A61K 31/216, A61K 31/7028, A61K 35/00, A61P 37/02, A61P 1/00

(54) **HERSTELLUNG EINER VIABLEN, LAGERFÄHIGEN WURMEIERSUSPENSION**
PRODUCTION OF A VIABLE, STORABLE WORM EGG SUSPENSION
PRÉPARATION D'UNE SUSPENSION D' OEUFS D'HELMINTHES STABLE ET VIABLE

(30) Priorität: 02.04.2007 EP 07006838
(43) Veröffentlichungstag der Anmeldung: 09.12.2009
(73) Patentinhaber: Dr. Falk Pharma Gmbh, 79108 Freiburg (DE)
(72) Erfinder: WILHELM, Rudolf, 76476 Bischweier (DE); ROEPSTORFF, Allan, Knud, DK-2610 Rødovre (DK); KAPEL, Christian, Moliin, Outzen, DK-2960 Rungsted Kyst (DK)
(74) Vertreter: Keller, Günter
(86) Internationale Anmeldenummer: PCT/EP2008/002542
(87) Internationale Veröffentlichungsnummer: WO 2008/119534

(56) Entgegenhaltungen:
- EP-A- 1 530 972
- WO-A-2007/076868
- DE-A1- 10 163 115
- SUMMERS R W ET AL: "Trichuris suis therapy in Crohn's disease" GUT, Bd. 54, Nr. 1, Januar 2005 (2005-01), Seiten 87-90, XP002449733 ISSN: 0017-5749 in der Anmeldung erwähnt
- SUMMERS ROBERT W ET AL: "Trichuris suis seems to be safe and possibly effective in the treatment of inflammatory bowel disease." AMERICAN JOURNAL OF GASTROENTEROLOGY, Bd. 98, Nr. 9, September 2003 (2003-09), Seiten 2034-2041, XP002449734 ISSN: 0002-9270 in der Anmeldung erwähnt
- BOES J ET AL: "Embryonation and infectivity of Ascaris suum eggs isolated from worms expelled by pigs treated with albendazole, pyrantel pamoate, ivermectin or piperazine dihydrochloride" VETERINARY PARASITOLOGY, Bd. 75, Nr. 2-3, 28. Februar 1998 (1998-02-28), Seiten 181-190, XP002449735 ISSN: 0304-4017
- ELLIOTT D ET AL: "Helminthic parasite exposure protects mice from colitis" DIGESTIVE AND LIVER DISEASE, Bd. 32, Nr. Supplement 1, Mai 2000 (2000-05), Seite A26, XP009088955 & INTERNATIONAL MEETING ON INFLAMMATORY BOWEL DISEASES; CAPRI, ITALY; JUNE 18-21, 2000
- HUNTER M M ET AL: "Review article: Helminths as therapeutic agents for inflammatory bowel disease" ALIMENTARY PHARMACOLOGY & THERAPEUTICS, BLACKWELL SCIENTIFIC PUBLICATIONS LTD., CAMBRIDGE, GB, Bd. 19, Nr. 2, 15. Januar 2004 (2004-01-15), Seiten 167-177, XP002442711 ISSN: 0269-2813
- REDDY A ET AL: "The use of Trichuris suis and other helminth therapies to treat Crohn's disease" PARASITOLOGY RESEARCH, SPRINGER VERLAG, BERLIN, DE, Bd. 100, Nr. 5, April 2007 (2007-04), Seiten 921-927, XP002442714 ISSN: 0932-0113

## Beschreibung

Die vorliegende Erfindung betrifft ein Reinigungsverfahren zur Herstellung von viablen, entwicklungsfähigen Suspensionen, die Eier von parasitären Helminthen enthalten, und zur therapeutischen Anwendung geeignet sind.

Der Einfluss von Parasiteninfektionen auf die Aktivierung des Immunsystems ihrer tierischen Wirte ist bekannt (Review, D M McKay, Parasitology 2006, 132: 1-12). Von dieser Aktivierung wird auch das Vorkommen und der Verlauf von Krankheiten des Immunsystems beeinflusst. Epidemiologische Studien zeigen, dass in Regionen mit hohen Raten von Wurminfektionen Autoimmunerkrankungen seltener sind, als in Regionen in denen auf Grund besserer hygienischer Verhältnisse diese Infektionsraten niedriger sind. Cytokinprofile von Patienten mit Morbus Crohn, einer chronisch entzündlichen Darmerkrankung, haben gezeigt, dass Th2-Immunzellen durch Helminthen Infektionen stimuliert werden können. Morbus Crohn, eine Th1-dominierte Autoimmunerkrankung kann durch eine Helminthen Infektion verhindert bzw. beeinflusst werden (Summers et al., Am J Gastroenterol 2003, 98: 2034-2041). Auch andere durch Th1-Immunzellen hervorgerufene Erkrankungen, wie auch durch Helicobacter verursachte Gastritis und autoimmune Enzephalomyelitis sind beeinflussbar.

Schon 1973 berichtete Beer (Parasitology 1973, 67: 253-262), dass *Trichuris suis* ein geeigneter Nematode sein könnte, um eine Immunisierung beim Menschen zu erreichen, ohne dass eine pathogene Nematodeninfektion erfolgt. *Trichuris suis* ist eng verwandt mit *Trichuris trichiura* und überlebt im menschlichen Gastrointestinaltrakt ohne sich jedoch zu vermehren. Therapeutische Maßnahmen sind während dieser selbst limitierten Infektion nicht notwendig. Eine Infektion durch *Trichuris suis* kann daher eine Immunisierung bewirken.

Hunter et al., Aliment.Pharmacol.Ther. 2004, S. 167-177 beschreiben die Verwendung von Helminthen als Therapeutika für entzündliche Darmerkrankungen. Reddy et al., Parasitol.Res. (2007), S. 921-927 beschreiben die Verwendung von *Trichuris suis* für die Behandlung von Morbus Crohn.

R.J.S. Beer (Parasitology 1972, 65: 343-350) beschreibt eine Methode zum Sammeln und Reinigen von Wurmeiem. Wurmeier im embryonisierten Stadium werden in einer 0.2%igen Kaliumdichromatlösung bei 32°C kultiviert und täglich belüftet. In DE 101 63 115 wird eine ähnliche Reinigungsmethode beschrieben. Es werden Wurmeier mit Hilfe von Hydroxyl- oder Sauerstoffradikalen produzierenden chemischen Reaktionen von eventuell vorhandenen Mikroorganismen und Viren befreit. Besonders die sogenannte Fenton-Reaktion wird beschrieben. Bei dieser entsteht unter Einfluss von FeCl₂ aus H₂O₂ Sauerstoff in statu nascendi, dessen desinfizierende Wirkung genutzt wird.

Boes et al., Veterinary Parasitology (1998), Seite 181-190 beschreiben die Embryonierung und die Infektiosität von Eiern des Wurmes *Ascaris suum,* die aus weiblichen Würmern gewonnen wurden und in Schwefelsäure embryonisiert wurden.

Summers und Mitarbeiter (GUT 2005, 54: 87-90) reinigen T. suis-Eier ebenfalls mit einer 0.2%igen Dichromatlösung in Phosphatpuffer bei pH 6-7, nachdem sie zunächst 5-6 Wochen in einer Pufferlösung, die Antibiotika (Penicillin/Streptomycin/Amphotericin B) enthält, embryonisiert wurden.

Die Verwendung solcher Bakterien und Viren abtötender Agentien hat verschiedene wesentliche Nachteile:
Sowohl bei der Verwendung einer Dichromatlösung wie auch bei der Verwendung einer Fentonlösung muss diese nach kurzer Zeit entfernt werden, um eine Schädigung der Wurmeier zur vermeiden. Versuche zeigten zudem, dass während der nach der Reinigung erfolgenden Embryonisierung (3 Monate bei 22°C - 25°C) trotz dieser vorherigen Reinigung, eine starke Verkeimung auftritt. Auch während der anschließenden Lagerung der embryonisierten Eier bei 2-8°C kann eine Verkeimung nicht vermieden werden. Daher ist es wahrscheinlich, dass nach Entfernung solcher Mikroorganismen und Viren abtötender Agentien ein erneutes Wachstum von Keimen in den T. suis-Eisuspension erfolgt.

Zudem ist die restlose Entfernung von Dichromat aus der Lösung unbedingt notwendig. Dies ist jedoch sehr aufwendig.

Der Zusatz von Antibiotika zur Vermeidung einer starken Verkeimung im Embryonisierungsstadium der Eier wie von Summers beschrieben, verhindert zwar ein starkes Keimwachstum in diesem Stadium, ist aber nicht in der Lage, die Keime sicher abzutöten. Nach der Entfernung von Antibiotika in den nächsten Reinigungsschritten und der Lagerung bis zur Anwendung tritt erneut starkes Keimwachstum auf.

Die WO 2007/076868 beschreibt Zusammensetzungen mit Eiern von helminthischen Parasiten, die mit Schwefelsäure im Bereich von pH 0 bis 2 behandelt wurden.

Eine Aufgabe der vorliegenden Erfindung ist es, eine embryonierte Wurmeiersuspension herzustellen, die von kontaminierenden Viren und Mikroorganismen, insbesondere Bakterien gereinigt wird und die ohne Wachstum von Pilzsporen und Hefen gelagert werden kann. Die hier beschriebenen Verfahren erhalten die Entwicklungsfähigkeit der Wurmeier zu adulten Würmern. Die Wurmeiersuspensionen sind für eine therapeutische Anwendbarkeit geeignet.

Im Rahmen der vorliegenden Erfindung wurde überraschend gefunden, dass sich zur Lösung der beschriebenen Aufgabe ein kombiniertes Reinigungs- und Konservierungsverfahren besonders eignet, da für dieses Verfahren im Gegensatz zu den bisher bekannten Verfahren keine toxischen Substanzen (z.B. Kaliumdichromat, K2Cr₂O₇) und keine Desinfektionsmittel verwendet werden müssen.

Während des Reinigungsverfahren werden isolierte Eier von Helminthen, vorzugsweise von T. suis, in einer Säurelösung, vorzugsweise eine H₂SO₄-Lösung, mit einem pH-Wert ≤ 2 für die Dauer von 1-72 Std. inkubiert (siehe Beispiel 1). Die optimale Inkubationszeit ist 2 bis 8 Stunden, bevorzugt 3 Stunden. Die Inkubation erfolgt bei einer Temperatur von 4°C bis 37°C, bevorzugt 25°C bis 35°C.

Bei dem Verfahren zur Herstellung einer oral verabreichbaren pharmazeutischen Präparation enthaltend eine lagerfähige Suspension von lebensfähigen Eiern von parasitären, nicht human pathogenen Helminthen, bei der sich nach Einnahme eine ausreichende Zahl an Helminthen entwickelt, die zu einer Stimulation regulatorischer humaner T-Zellen führt, wird zunächst die Suspension der Helmintheneier in einem ersten Schritt einer Säurebehandlung bei einem pH-Wert von ≤ 2 unterzogen. In einem weiteren Schritt wird der pH-Wert auf ≥ 4 angehoben und ein pharmakologisch annehmbares Konservierungsmittel zugefügt. Grundsätzlich ist es auch möglich, zuerst das Konservierungsmittel zuzugeben und dann die Säurebehandlung durchzuführen. Bevorzugt wird aber zuerst die Säurebehandlung durchgeführt und dann das Konservierungsmittel zugegeben.

Bevorzugt wird die Säurebehandlung durch Zugabe von Schwefelsäure bewirkt. Bei der Säurebehandlung wird eine ausreichende Menge an Säure zugegeben, um den pH-Wert in einen Bereich zwischen etwa 0,5 und 2 abzusenken. In einer weiteren Ausführungsform wird der pH-Wert auf etwa 0 bis <1 abgesenkt. Geeignete Säuren sind Salzsäure, Salpetersäure oder bevorzugt Schwefelsäure. Die Säurebehandlung darf nur über einen verhältnismäßig kurzen Zeitraum erfolgen, damit die Wurmeier nicht zu stark geschädigt werden. Hier besteht auch ein Zusammenhang zwischen Konzentration an Säure und Dauer der Säurebehandlung. Bevorzugt wird der pH-Wert auf weniger als 2, besonders bevorzugt sogar auf pH 0,6 bis 0,8, abgesenkt. Die Dauer der Säurebehandlung beträgt einige Minuten bis wenige Stunden, bevorzugt 120 Minuten bis 360 Minuten. Der noch tolerierbare kurzfristige Zeitraum schließt den Prozess der Embryonierung der Wurmeier (3 Monate bei 22 - 25°C) mit ein.

Nach der Säurebehandlung wird der pH-Wert durch Zugabe einer geeigneten Base, beispielsweise NaOH, wieder auf einen pH-Wert > 4 erhöht. Anschließend wird ein oder mehrere Konservierungsmittel zugegeben, wobei grundsätzlich sämtliche für die Konservierung von Lebensmitteln oder Pharmazeutika geeignete Stoffe eingesetzt werden können. Bevorzugt werden solche Konservierungsmittel eingesetzt, die gerade von Patienten die an Morbus Crohn leiden, gut vertragen werden.

Bevorzugt wird das Konservierungsmittel ausgewählt aus der Gruppe bestehend aus Sorbinsäure, Benzoesäure, Salzen dieser Säuren, Parabenzoesäureester, p-Hydroxy-Benzoesäureester, Propylenglykol oder Kombinationen dieser Konservierungsmittel.

Besonders bevorzugt eingesetzt wird ein oder mehrere der Konservierungsmittel ausgewählt aus der Gruppe bestehend aus
Sorbinsäure in einer Konzentration von 0,01, bis 0,2 %, insbesondere 0,1 bis 0,2 %, Benzoesäure in einer Konzentration von 0,1 bis 0,3 Gew.-%, p-Hydroxy-Benzoesäureester in einer Konzentration von 0,02 bis 0,3 Gew.-%, Propylenglykol in einer Konzentration von 5 bis 20 Gew.-% oder eine Kombination der genannten Konservierungsmittel in den angegebenen Konzentrationsbereichen.

Alternativ zu starken Säuren eignen sich auch schwächere saure Lösungen unter Zusatz der genannten Konservierungsmittel, bevorzugt Sorbinsäure und seine Salze, zur raschen Inaktivierung von Mikroorganismen und Viren. Die Verwendung dieser Lösungen hat gegenüber der Verwendung von starken Säuren jedoch den Vorteil, dass die Wurmeier auf Grund des weniger sauren pH-Wertes in einem einzigen Medium aufgereinigt, embryonisiert (3 Monate bei 22 - 25°C) gelagert und am Patienten angewendet werden können. Diese Langzeitanwendung führt zu keiner Beeinträchtigung der Lebensfähigkeit der Wurmeier (siehe Beispiel 5). Dieses schonende Verfahren verwendet konservierungsmittelhaltige Medien mit einem pH-Wert von ≥ 1, bevorzugt ≤ 2, und führt zu keinen Einschränkungen hinsichtlich der mikrobiologischen Reinheit der Wurmeisuspensionen.

Die resultierenden Konservierungsmittel-enthaltenden Suspensionen mit embryonierten Wurmeiern eignen sich zur Anwendung am Patienten als Trinklösung. Diesen Wurmeisuspensionen können gegebenenfalls weitere pharmazeutisch verträgliche Zusatzstoffe wie Farbstoffe, Geschmacksstoffe oder Verdickungsmittel zugefügt werden.

Gegenstand der Erfindung sind daher oral verabreichbare pharmazeutische Präparationen enthaltend eine lagerfähige Suspension von lebensfähigen Eiern von parasitären, nicht human pathogenen Helminthen, insbesondere *T*. *suis* wobei sich nach Einnahme der Suspension eine ausreichende Zahl an Helminthen entwickelt, die zu einer Stimulation regulatorischer T-Zellen führt, und wobei die Suspension weniger als 1000 Kolonie-bildende (cfu = colony forming unit) Mikroorganismen pro ml Suspension beinhaltet. Bevorzugt enthält die oral verabreichbare pharmazeutische Präparation weniger als 100 Kolonie-bildende Einheiten an Mikroorganismen pro ml Suspension. Besonders bevorzugt enthält die verabreichbare pharmazeutische Präparation weniger als 10 Kolonie-bildende Einheiten an Mikroorganismen pro ml Suspension. Die Anzahl der Kolonie-bildenden Einheiten wird mit üblichen mikrobiologischen Verfahren bestimmt.

Unter Mikroorganismen versteht man hier Bakterien, Viren, Pilze, Hefen und Protozoen, wobei solche Mikroorganismen nicht vorhanden sein dürfen, die nachteilige gesundheitliche Auswirkungen haben können.

Durch das erfindungsgemäße Verfahren ist es möglich, die Suspension von lagerfähigen Eiern von parasitären, nicht humanpathogenen Helminthen in einer Form bereitzustellen, die für die pharmazeutische Verwendung geeignet sind. Durch die erfindungsgemäße Behandlung werden einerseits kontaminierende Mikroorganismen, insbesondere Bakterien, aber auch Pilze, Viren sowie gegebenenfalls Protozoen so weit abgetötet, dass das Präparat zum pharmazeutischen Einsatz geeignet ist. Andererseits ist aber die Behandlung so schonend, dass die Wurmeier ihre Fähigkeit beibehalten, nach Einnahme im Darm des Patienten heranzuwachsen, damit die gewünschte Stimulierung des Immunsystems erfolgen kann.

Die erfindungsgemäßen Präparate sind insbesondere zur Behandlung von verschiedenen entzündlichen Darmerkrankungen, vor allem chronisch entzündlichen Darmerkrankungen geeignet. In besonders vorteilhafter Weise können die Präparate zur Behandlung von entzündlichen Darmerkrankungen eingesetzt werden, die als Morbus Crohn bezeichnet werden.

### Beispiel 1: Abreicherung von Keimen in einer T. suis - Eisuspension:

In einer *Trichuris suis* Eisuspension (TSO) in Phosphatpuffer (pH 7) in einer Konzentration von 2.400 Eiern/ml wurde nach den Angaben im DAB (deutsches Arzneibuch, Abschnitt 2.6.12, zuletzt aktualisiert mit der 24. Ergänzungslieferung in 2006) die Keimzahl bestimmt. Es wurde ein Keimstatus von 190 000 cfu/ml gefunden. Diese Suspension wurde dann mit verdünnter H₂SO₄-Lösung auf pH 2 umgepuffert. Nach 3 h Lagerung bei 30°C war die Keimzahl auf < 20 cfu/ml reduziert worden.

Die Güte des vorgestellten Verfahrens wurde dann überprüft. Hierzu wurden definierte Teststämme von Bakterien, Hefen und Pilzen nach den Vorgaben des deutschen Arzneibuches (DAB) verwendet. In den Beispielen 2a, 2b und 2c sind die Ergebnisse dieser Prüfung dargestellt.

### Beispiel 2: Bestimmung der Keimzahl

a. Eine Nährsuspension entsprechend DAB 2.6.13 wurde mit den in Tab. 1 vorgegebenen sporenbildenden und nicht-sporenbildenden Testbakterien beimpft. Anschließend wurde mit einer verdünnten H₂SO₄-Lösung auf pH 2 umgepuffert und 6 h bei 30°C gelagert. Die darauf folgende Keimzahlbestimmung ergab die Werte wie in Tab. 1 dargestellt.

**Tab. 1**

| Keim | T₀ | H₂SO₄ pH 2 nach 6 h |
|---|---|---|
| *Clostridium sporog.* | 8 000 000 | < 10 |
| *Bacillus subtilis* | 1 400 000 | 20* |
| *Escherichia coli* | 130 000 | < 10 |
| *Staphylococus aureus* | 800 000 | < 10 |
| *Salmonella typhymurium* | 250 000 | < 10 |

| | | |
|---|---|---|
| * Durch eine längere Behandlung mit Säure für ca. 6 bis 10 Stunden konnte die Anzahl der Kolonie-bildenden Einheiten auf weniger als 10 reduziert werden. | | |

b. Eine Nährsuspension entsprechend DAB 2.6.13 wurde mit den in Tab. 2 vorgegebenen Testhefen beimpft. Anschließend wurde mit einer verdünnten H₂SO₄-Lösung auf pH 2 bzw. pH 1 umgepuffert und 3 h bzw. 48 h bei 25°C gelagert. Die darauf folgende Bestimmung ergab die Werte wie in Tab. 2 dargestellt.

**Tab. 2**

| Keim | T₀ | T₃ₕ | T₄₈ₕ | T₃ₕ | T₄₈ₕ |
|---|---|---|---|---|---|
| | | H₂SO₄ pH 2 | | H₂SO₄ pH 1 | |
| Saccharomyces cer. | 470 000 | 380 000 | 220 000 | 39 000 | 40* |
| Candida albicans | 690 000 | 380 000 | 32 000 | 26 000 | <10 |

| | | | | | |
|---|---|---|---|---|---|
| * Durch eine längere Behandlung mit Säure bis zu einer Dauer von 72 Stunden konnte die CfU auf 5 reduziert werden. | | | | | |

c. Eine Nährsuspension entsprechend DAB 2.6.13 wurde mit den in Tab. 3 vorgegebenen Testschimmel beimpft. Anschließend wurde mit einer verdünnten H₂SO₄-Lösung auf pH 2, pH 1 und pH 0 umgepuffert und 3 h bzw. 48 h bei 25°C gelagert. Die darauf folgende Bestimmung ergab die Werte wie in Tab. 3 dargestellt.

**Tab. 3**

| Keim | T₀ | T₃ₕ | T₄₈ₕ | T₃ₕ | T₄₈ₕ | T₃ₕ | T₄₈ₕ |
|---|---|---|---|---|---|---|---|
| | | H₂SO₄ pH 2 | | H₂SO₄ pH 1 | | H₂SO₄ pH 0 | |
| Penicillium | 490 000 | 90 000 | 32 000 | 39 000 | 14 000 | 70 | <10 |
| brevicomp. | | | | | | | |
| Aspergillus | 800000 | 370000 | 700000 | 500000 | 500000 | 50 | <10 |
| niger | | | | | | | |

Wenn durch die Säurebehandlung die CfU bei bestimmten Keimen nicht hinreichend reduziert wurde, wurde die Dauer der Säurebehandlung entsprechend verlängert auf bis zu 72 Stunden.

Die Beispiele 1 und 2 a-c zeigen, dass nach kurzer Zeit Bakterien mit einer verdünnten H₂SO₄-Lösung pH 2 effektiv abgetötet werden. Hefen und Pilze benötigen entweder eine längere Inkubationszeit oder stärker saure pH-Werte um effektiv abgetötet zu werden.

Die beschriebenen Reinigungsschritte sind überraschend auch zur Herstellung einer virenfreien *T*. *suis*-Eisuspension-geeignet.

### Beispiel 3: Abreicherung von Viren:

Inaktivierung von Murine Leukemia Virus (MuLV), Pseudorabies Virus (PRV), Porcine Parvovirus (PPV) und Feline Calicivirus (FCV) bei der Herstellung von *Trichuris suis* Eisuspension (TSO). Es handelt sich bei den eingesetzten Viren um Modellviren.

Der Virustiter in den beimpften Proben wurde durch Endpunkttitration bestimmt. In diesem an sich bekannten Verfahren werden verdünnte Proben auf Mikrotiterplatten, die leicht zu indentifizierende Indikatorzellen enthalten, aufgebracht. Nach dieser Infektion der Indikatorzellen mit den virushaltigen Proben verändert sich durch die Zell-Lysis die Morphologie der Zellen. Diese Zell-Lyse ist unter dem Mikroskop leicht zu messen.

Die getesteten Viren, umhüllte und nicht-umhüllte, wurden in hoher Konzentration in eine 0.01 N H₂SO₄-Lösung (pH 2, 30°C), die außerdem TSO enthält, gegeben. Nach 10 min., 3 h und 72 h wurde die Aktivität der Viren bestimmt. Nach 10 min. waren die umhüllten Viren PRV und MuLV, nach 3 h die nicht-umhüllten Viren FCV und PPV nicht mehr infektiös. Die Verwendung einer mit 1 N NaOH neutralisierten Gegenprobe zeigte auch nach-3 h Inkubation, dass alle 4 Viren noch virulent waren. Wegen der Cytotoxizität einer 0,2%igen Kaliumdichromatlösung in 0,01 N Schwefelsäure war eine Testung dieser Lösung als Vergleich nicht möglich.

Nur lange Inkubation und die Absenkung des pH-Wertes auf einen pH-Wert von 0, bevorzugt <1, ermöglichten eine deutliche Reduktion auch von Pilzen und Hefen (siehe Beispiel 2, Tabelle 2 und 3). Dieses bereits neue Verfahren der Aufreinigung in sehr saurem Medium hat den Nachteil, dass die Überlebensfähigkeit einer Wurmeiersuspension beeinträchtigt wird.

Als zweites Merkmal der vorliegenden Erfindung wurde nun überraschend gefunden, dass durch die Addition von oral verträglichen zur Konservierung verwendeten chemischen Substanzen der Embryonierungskoeffizient erhalten werden konnte. Der Embryonierungskoeffizient ist ein eindeutiger Marker für die Überlebensfähigkeit von Wurmeiersuspensionen. Zudem ermöglichte die Verwendung von Konservierungsmitteln die Inaktivierung von kontaminierenden Mikroorganismen schon bei pH 4. Als bevorzugte oral-verträgliche Konservierungsmittel haben sich Benzoesäure, Sorbinsäure, deren Salze und Propylenglykol herausgestellt (siehe Beispiel 4 a-e).

Erst der Zusatz von Konservierungsmitteln ermöglicht die Verwendung von wesentlich weniger sauren und damit für die Wurmeier verträglicheren Medien. Auf diese Weise können die Wurmeier in einem einzigen Medium aufgereinigt, embryoniert, gelagert und am Patienten zur, Anwendung gebracht werden. Somit steht ein Medium für die Langzeitanwendung zur Verfügung. Eine mögliche Rekontamination der TSO-Suspensionen mit neuen Keimen kann durch die Verwendung verdünnter Säuren und dem Zusatz von Konservierungsmittel verhindert werden. Das Wachstum von neuen Keimen wird durch das beschriebene kombinierte Verfahren der Inaktivierung und Konservierung einer TSO-Suspension vermieden. Damit hat dieses äußerst schonende Verfahren eindeutige Vorteile gegenüber dem Verfahren, das auf den Einsatz von starken Säuren beruht.

### Beispiel 4: Bestimmung der Koloniezahl verschiedener Mikroorganismen

Anheben des pH-Wertes von pH 2 auf pH 4 und Zusatz von Konservierungsmitteln

Versuchsbedingungen für die Beispiele 4a bis 4e:
Die getesteten Proben enthielten je 10.000 TSO/ml (± 10 %) in Phosphat-Puffer von pH 4 und entweder 0,2 % Benzoesäure, 0,1 % Sorbinsäure oder 15 % Propylenglykol.

Den Testproben wurden folgende Keime zugesetzt:
- Aspergillus niger als Einzelkeim
- Pseudomonas aruginosa, Staphylococcus aureus, Candida albicans und Escherichia coli in Kombination.

Die Inkubationstemperatur betrug 25°C. Die Testplatten wurden nach 1 bis 7 Tagen ausgewertet. Es wurden folgende Ergebnisse erhalten:

| 4 a Pseudomonas aeruginosa | | | |
|---|---|---|---|
| Konservierungsmittel | T 0 (cfu/ml) | T 1d (cfu/ml) | T 7d (cfu/ml) |
| Benzoesäure 0,2% | 180.000 | < 10 | < 10 |
| Sorbinsäure 0,1% | 180.000 | 30 | < 10 |
| Propylenglykol 15% | 180.000 | 10 | < 10 |

| 4 b Staphylococcus aureus | | | |
|---|---|---|---|
| Konservierungsmittel | T 0 (cfu/ml) | T 1d (cfu/ml) | T 7d (cfu/ml) |
| Benzoesäure 0,2% | 130.000 | < 10 | < 10 |
| Sorbinsäure 0.1% | 130.000 | 240 | < 10 |
| Propylenglykol 15% | 130. 000 | 14.000 | < 10 |

| 4 c Escherichia coli | | | |
|---|---|---|---|
| Konservierungsmittel | T 0 (cfu/ml) | T 1d (cfu/ml) | T 7d (cfu/ml) |
| Benzoesäure 0,2% | 140.000 | < 10 | < 10 |
| Sorbinsäure 0, 1 % | 140.000 | 490 | <10 |
| Propylenglykol 15% | 140.000 | 7.100 | < 10 |

| 4 d Candida albicans | | | |
|---|---|---|---|
| Konservierungsmittel | T 0 (cfu/ml) | T 1d (cfu/ml) | T 7d (cfu/ml) |
| Benzoesäure 0,2% | 240.000 | < 10 | < 10 |
| Sorbinsäure 0,1% | 240.000 | 190 | < 10 |
| Propylenglykol 15% | 240.000 | 8.600 | 30 |

| 4 e Aspergillus niger | | | |
|---|---|---|---|
| Konservierungsmittel | T 0 (cfu/ml) | T 1d (cfu/ml) | T 7d (cfu/ml) |
| Benzoesäure 0,2% | 120.000 | 90 | < 10 |
| Sorbinsäure 0,1% | 120.000 | 20.000 | < 10 |
| Propylenglykol 15% | 120.000 | 30.000 | 9.000 |

Wenn eine starke Kontamination mit Pilzen vorliegt, werden bevorzugt Sorbinsäure, Benzoesäure und/oder p-Hydroxy-Benzoesäureester eingesetzt oder Propylenglykol mit diesen Konservierungsstoffen kombiniert eingesetzt.

Ein weiterer Vorteil der kombinierten Inaktivierung von Mikroorganismen mittels verdünnter Säuren und der Verwendung von Konservierungsmitteln ist, dass während der weiteren Verwendung der TSO-Suspension eine mögliche Rekontaminierung mit neuen Keimen, verhindert werden kann. Das Wachstum von neuen Keimen wird durch das beschriebene kombinierte Verfahren der Inaktivierung und Konservierung einer TSO-Suspension vermieden.

Die Vorbereitung zur weiteren Herstellung und Anwendung beim Patienten erfordert eine Anhebung des pH-Wertes auf einen Bereich von pH 2 - 7, vorzugsweise pH 4 - 6. Auch für diesen dritten Schritt ist die Addition von geeigneten Konservierungsmitteln erforderlich. Zu verwendende Konservierungsmittel müssen für die orale Anwendung geeignet sein. Als geeignet haben sich hierbei Sorbinsäure in einer Konzentration von 0,1 - 0,2%, Benzoesäure in einer Konzentration von 0,1 - 0,3%, p-Hydroxy-Benzoesäureester in Konzentrationen von 0,02 - 0,3%, oder Propylenglykol in einer 5 - 20 %igen wässrigen Lösung erwiesen. Ebenfalls geeignet sind Gemische der oben genannten Konservierungsmittel.

Überraschend wurde gefunden, dass durch dieses Reinigungsverfahren die Viabilität der Wurmeiersuspension nicht beeinträchtigt wird.

### Beispiel 5: Lebensfähigkeit der Wurmeier

Nicht-embryonierte Wurmeier in einer Konzentration von 40'000 Eiern/ml wurden in unterschiedlichen Medien suspendiert und embryoniert (22 - 25°C). Nach einer Inkubationsdauer von 60 und 90 Tagen wurden die einzelnen Suspensionen mikroskopisch untersucht und der Embryonierungskoeffizient aus der Gesamtanzahl an Wurmeiern sowie der Anzahl an morphologisch intakten und embryonierten Wurmeiern berechnet. Der Embryonierungskoeffizient (EK) ist dabei ein Indikator für die Lebensfähigkeit der Wurmeier. Zu Beginn der Embryonierung liegen nur nicht embryonierte Wurmeier vor, so dass der Koeffizient Null beträgt. Er nimmt während der Dauer der Embryonierung zu und erreicht ab Tag 60 bei den gewählten Inkubationsbedingungen sein Maximum. In der Regel werden Werte um die 90% erzielt.

| **Suspensionsmedium** | | | | **EK Tag 0** | **EK Tag 60** | **EK Tag 90** |
|---|---|---|---|---|---|---|
| Schwefelsäure pH Kaliumsorbat | 1 | + | 0.01% | 0 | 0,91 | 0,91 |
| Schwefelsäure pH Kaliumsorbat | 1 | + | 0.07% | 0 | 0,93 | 0,91 |
| Schwefelsäure pH 0,8 | | | | 0 | 0,94 | 0,90 |
| Schwefelsäure pH 0,5 | | | | 0 | 0,92 | 0,77 |

Für alle Medien konnte nach einer Embryonierungsdauer von 60 Tagen ein Anteil an embryonierten Eiern von über 90% gefunden werden. Während die Konservierungsmittel-enthaltenden Medien unabhängig von der Konzentration des Konservierungsmittels nach weiteren 30 Tagen keine Abnahme des Embryonierungskoeffizienten zeigten, ist bei der Schwefelsäure pH 0,5 bereits ein Abfall des Anteils der embryonierten Wurmeier zu verzeichnen. Der Zusatz von oral verträglichen Konservierungsmittel, bevorzugt Sorbinsäure und seine Salze, erlaubt damit die Anwendung von für die Wurmeier unschädlichen pH-Werten. Dieses Verfahren kann daher zur Herstellung einer Präparation, geeignet für eine stabile Lagerung, eingesetzt werden (schonendes Verfahren).

Die Vorbereitung zur weiteren Herstellung erfordert eine Anhebung des pH-Wertes auf einen Bereich von pH 2 - 7, vorzugsweise pH 4-6, sowie die Addition von geeigneten Konservierungsmitteln. Zu verwendende konservierungsmittel müssen für die orale Anwendung geeignet sein. Als geeignet haben sich hierbei Sorbinsäure in einer Konzentration von 0,01 - 0,2%, Benzoesäure in einer Konzentration von 0,1 - 0,3%, oder Propylenglykol in einer 5 - 20%igen wässrigen Lösung erwiesen. Ebenfalls geeignet sind Gemische der oben genannten Konservierungsmittel.

## Patentansprüche

1. Verfahren zur Herstellung einer oral verabreichbaren pharmazeutischen Präparation enthaltend eine lagerfähige Suspension von lebensfähigen Eiern von parasitären, nicht humanpathogenen Helminthen, nämlich von *Trichuris suis,* wobei sich nach Einnahme der Suspension eine ausreichende Zahl an Helminthen entwickelt, die zu einer Stimulation regulatorischer humaner T-Zellen führt, **dadurch gekennzeichnet, dass** die Suspension der Helmintheneier in einem Schritt einer Säurebehandlung bei einem pH-Wert von ≤ 2 unterzogen wird und, dass in einem weiteren Schritt der pH-Wert auf ≥ 4 eingestellt wird und ein pharmakologisch annehmbares Konservierungsmittel zugefügt wird.

2. Verfahren nach Anspruch 1, **dadurch gekennzeichnet, dass** die Säurebehandlung durch Zugabe von Schwefelsäure bewirkt wird.

3. Verfahren nach einem der vorhergehenden Ansprüche, **dadurch gekennzeichnet, dass** das Konservierungsmittel ausgewählt ist aus der Gruppe bestehend aus Sorbinsäure, Benzoesäure, Salzen dieser Säuren, p-Benzoesäureester, Propylenglykol oder eine Kombination dieser Konservierungsmittel.

4. Verfahren nach Anspruch 3, **dadurch gekennzeichnet, dass** eines oder mehrere der Konservierungsmittel ausgewählt aus der Gruppe bestehend aus Sorbinsäure in einer Konzentration von 0,01 bis 0,2 Gew.-%, Benzoesäure in einer Konzentration von 0,1 bis 0,3 Gew.-%, p-Hydroxy-Benzoesäureester in einer Konzentration von 0,02 bis 0,3 Gew.-%, Propylenglykol in einer Konzentration von 5 bis 20 Gew.-% oder eine Kombination der genannten Konservierungsmittel in den angegebenen Konzentrationsbereichen zugegeben wird.

5. Verfahren nach einem der vorhergehenden Ansprüche, **dadurch gekennzeichnet, dass** der Suspension gegebenenfalls weitere pharmazeutisch verträgliche Zusatzstoffe zugefügt werden.

6. Oral verabreichbare pharmazeutische Präparation enthaltend eine lagerfähige Suspension von lebensfähigen Eiern von parasitären, nicht humanpathogenen Helminthen, nämlich *Trichuris suis,* wobei sich nach Einnahme der Suspension eine ausreichende Zahl an Helminthen entwickelt, die zu einer Stimulation regulatorischer T-Zellen führt, wobei die Suspension weniger als 1000 Kolonie-bildende Mikroorganismen pro ml Suspension beinhaltet, **dadurch gekennzeichnet, dass** sie nach einem der Verfahren gemäß Anspruch 1 bis 5 herstellbar ist.

7. Oral verabreichbare pharmazeutische Präparation nach Anspruch 6, **dadurch gekennzeichnet, dass** die Suspension weniger als 100 Kolonie-bildende Einheiten an Mikroorganismen pro ml Suspension enthält.

8. Oral verabreichbare pharmazeutische Präparation nach Anspruch 7, **dadurch gekennzeichnet, dass** die Suspension weniger als 10 Kolonie-bildende Einheiten an Mikroorganismen pro ml Suspension enthält.

9. Oral verabreichbare pharmazeutische Präparation nach Anspruch 6 bis 8, **dadurch gekennzeichnet, dass** die Mikroorganismen ausgewählt sind aus der Gruppe bestehen aus Bakterien, Viren, Pilze, Hefen und Protozoen.

10. Verwendung einer lagerfähigen Suspension von Eiern parasitärer Helminthen, nämlich von *Trichuris suis,* hergestellt nach einem Verfahren gemäß einem der Ansprüche 1 bis 5 zur Herstellung einer pharmazeutischen Präparation zur Behandlung von entzündlichen Darmerkrankungen, insbesondere chronisch entzündlichen Darmerkrankungen.

11. Verwendung nach Anspruch 10, **dadurch gekennzeichnet, dass** es sich bei der entzündlichen Darmerkrankung um Morbus Crohn handelt.

## Claims

1. A process for the preparation of a pharmaceutical preparation for oral administration, comprising a storable suspension of viable eggs of parasitic helminths which are nonpathogenic to humans, viz. *Trichuris suis,* where, after the ingestion of the suspension, a sufficient number of helminths develops to result in a stimulation of regulatory human T-cells, **characterized in that** the suspension of the helminth eggs is subjected, in one step, to an acid treatment at a pH of ≤ 2 and **in that**, in a further step, the pH is adjusted to ≥ 4 and a pharmacologically acceptable preservative is added.

2. The process as claimed in claim 1, **characterized in that** the acid treatment is brought about by addition of sulfuric acid.

3. The process as claimed in any of the preceding claims, **characterized in that** the preservative is selected from the group consisting of sorbic acid, benzoic acid, salts of these acids, p-benzoic acid esters, propylene glycol, or a combination of these preservatives.

4. The process as claimed in claim 3, **characterized in that** one or more preservatives selected from the group consisting of
sorbic acid at a concentration of from 0.01 to 0.2% by weight,
benzoic acid at a concentration of from 0.1 to 0.3% by weight,
p-hydroxy-benzoic acid esters at a concentration of from 0.02 to 0.3% by weight,
propylene glycol at a concentration of from 5 to 20% by weight or a combination of the abovementioned preservatives in the abovementioned concentration ranges is added.

5. The process as claimed in any of the preceding claims, **characterized in that**, if appropriate, further pharmaceutically acceptable additives are added to the suspension.

6. A pharmaceutical preparation for oral administration, comprising a storable suspension of viable eggs of parasitic helminths which are nonpathogenic to humans, viz. *Trichuris suis,* where, after the ingestion of the suspension, a sufficient number of helminths develops to result in a stimulation of regulatory T-cells, the suspension comprising fewer than 1000 colony-forming microorganisms per ml of suspension, **characterized in that** it can be prepared by any of the processes as claimed in claims 1 to 5.

7. The pharmaceutical preparation for oral administration as claimed in claim 6, **characterized in that** the suspension comprises fewer than 100 colony-forming units of microorganisms per ml of suspension.

8. The pharmaceutical preparation for oral administration as claimed in claim 7, **characterized in that** the suspension comprises fewer than 10 colony-forming units of microorganisms per ml of suspension.

9. The pharmaceutical preparation for oral administration as claimed in claim 6 to 8, **characterized in that** the microorganisms are selected from the group consisting of bacteria, viruses, fungi, yeasts and protozoans.

10. The use of a storable suspension of eggs of parasitic helminths, viz. *Trichuris suis*, prepared by a process as claimed in any of claims 1 to 5 for making a pharmaceutical preparation for the treatment of inflammatory diseases of the intestine, in particular chronic-inflammatory diseases of the intestine.

11. The use as claimed in claim 10, **characterized in that** the inflammatory disease of the intestine is Crohn's disease.

## Revendications

1. Procédé de préparation d'une préparation pharmaceutique qu'on peut administrer par voie orale contenant une suspension apte au stockage d'oeufs viables d'helminthes parasitaires non pathogènes pour les humains, à savoir de *Trichuris suis,* un nombre suffisant d'helminthes se développant après la prise de la suspension, lesquels amènent une stimulation des cellules T humaines régulatoires, **caractérisé en ce que** la suspension des oeufs d'helminthes est soumise dans une étape à un traitement par acide à un pH ≤2 et **en ce que**, dans une autre étape, on règle le pH sur ≥ 4 et on ajoute un conservateur pharmacologiquement administrable.

2. Procédé selon la revendication 1, **caractérisé en ce que** le traitement à l'acide s'effectue par addition d'acide sulfurique.

3. Procédé selon l'une des revendications précédentes, **caractérisé en ce que** l'agent de conservation est sélectionné parmi le groupe composé de l'acide sorbique, de l'acide benzoïque, de sels de ces acides, de l'ester d'acide p-benzoïque, du propylèneglycol ou d'une combinaison de ces conservateurs.

4. Procédé selon la revendication 3, **caractérisé en ce qu'**on ajoute un ou plusieurs des conservateurs sélectionnés parmi le groupe composé des suivants:
acide sorbique dans une concentration de 0,01 à 0,2% en poids,
acide benzoïque dans une concentration de 0,1 à 0,3% en poids, ester d'acide p-hydroxy-benzoïque dans une concentration de 0,02 à 0,3% en poids, propylèneglycol dans une concentration de 5 à 20% en poids ou une combinaison des conservateurs mentionnés dans un intervalle de concentration donné.

5. Procédé selon l'une des revendications précédentes, **caractérisé en ce qu'**on ajoute à la suspension éventuellement d'autres additifs pharmaceutiquement compatibles.

6. Préparation pharmaceutique administrable par voie orale contenant une suspension apte au stockage d'oeufs viables d'helminthes parasitaires non pathogènes pour les humains, à savoir *Trichuris suis,* un nombre suffisant d'helminthes se développant après la prise de la suspension, lesquels amènent une stimulation des cellules T régulatoires, la suspension contenant moins de 1000 microorganismes formant des colonies par ml de suspension, **caractérisée en ce qu'**on peut la préparer selon l'une des procédés conformes aux revendications 1 à 5.

7. Préparation pharmaceutique administrable par voie orale selon la revendication 6, **caractérisée en ce que** la suspension contient par ml de suspension moins de 100 unités de microorganismes formant des colonies.

8. Préparation pharmaceutique administrable par voie orale selon la revendication 7, **caractérisée en ce que** la suspension contient par ml de suspension moins de 10 unités de microorganismes formant des colonies.

9. Préparation pharmaceutique administrable par voie orale selon la revendication 6 à 8, **caractérisée en ce que** les microorganismes sont sélectionnés parmi le groupe composé des bactéries, des virus, des champignons, des levures et des protozoaires.

10. Utilisation d'une suspension apte au stockage d'oeufs d'helminthes parasitaires, à savoir de *Trichuris suis,* préparés selon un procédé conforme à une des revendications 1 à 5 d'une préparation pharmaceutique pour le traitement de maladies inflammatoires de l'intestin, en particulier de maladies inflammatoires chroniques de l'intestin.

11. Utilisation selon la revendication 10, **caractérisée en ce qu'**il s'agit, pour la maladie de l'intestin inflammatoire, de Morbus Crohn.
